# FASCICULE DE BREVET EUROPEEN

(11) **EP 3 385 304 B1**
(45) Date de publication et mention de la délivrance du brevet: **15.07.2020**
(21) Numéro de dépôt: 18168305.3
(22) Date de dépôt: 18.12.2008
(51) Int. Cl.: C08G 77/08, C08L 83/04, C07C 49/92, C08K 5/56, C08J 3/24, C07F 3/06

(54) **COMPOSITION ORGANOPOLYSILOXANE DURCISSABLE A TEMPERATURE AMBIANTE EN UN ELASTOMERE**
BEI RAUMTEMPERATUR VULKANISIERBARE ORGANOPOLYSILOXAN-ZUSAMMENSETZUNG ZUR BILDUNG EINES ELASTOMERS
ORGANOPOLYSILOXANE COMPOSITION WHICH CAN BE VULCANISED AT AMBIENT TEMPERATURE IN ELASTOMER

(30) Priorité: 20.12.2007 FR 0708924; 02.09.2008 FR 0804801
(43) Date de publication de la demande: 10.10.2018
(62) Demande divisionnaire de: 08873016.3
(73) Titulaire: ELKEM SILICONES France SAS, 69003 Lyon (FR)
(72) Inventeur: MALIVERNEY, Christian, 69690 Saint Julien sur Bibost (FR); SAINT-JALMES, Laurent, 69390 Vourles (FR)

(56) Documents cités:
- EP-A- 0 354 138
- EP-A1- 1 964 872
- US-A1- 2007 203 316

## Description

La présente invention concerne un procédé requérant une composition organopolysiloxanique vulcanisable dès la température ambiante en élastomère réticulant par polycondensation et ne contenant pas de catalyseurs à base d'alkylétain qui présentent des problèmes de toxicité.

L'invention implique l'usage de catalyseurs de polycondensation dans la chimie des silicones et leurs utilisations comme catalyseurs de la réaction de polycondensation d'organopolysiloxanes.

Les formulations des élastomères réticulant par polycondensation font généralement intervenir une huile silicone, généralement un polydiméthylsiloxane, à terminaisons hydroxylées, éventuellement pré-fonctionnalisées par un silane de façon à présenter des extrémités alcoxy, un réticulant, un catalyseur de polycondensation, classiquement un sel d'étain ou un titanate d'alkyle, une charge de renfort et d'éventuels autres additifs comme des charges de bourrage, des promoteurs d'adhérence, des colorants, des agents biocides, etc.

Ces compositions organopolysiloxaniques vulcanisables dès la température ambiante sont bien connues et sont classées en 2 groupes distincts: les compositions monocomposantes (RTV-1) et les compositions bicomposantes (RTV-2).

Lors de la réticulation, l'eau (soit apportée par une humidité atmosphérique dans le cas des RTV-1, soit introduite dans une partie de la composition dans le cas des RTV-2) permet la réaction de polycondensation, qui conduit à la formation du réseau élastomérique.

Généralement, les compositions monocomposantes (RTV-1) réticulent quand elles sont exposées à l'humidité de l'air, c'est-à-dire qu'elles ne peuvent réticuler dans un milieu en confiné. Par exemple, les compositions silicones monocomposantes utilisées comme mastics ou adhésifs réticulent à froid suivant un mécanisme d'hydrolyse de fonctions réactives du type acétoxysilane, cétiminoxysilane, alcoxysilane..., suivi par des réactions de condensation entre des groupements silanols formés et d'autres fonctions réactives résiduelles. L'hydrolyse est généralement effectuée grâce à la vapeur d'eau qui diffuse dans le matériau à partir de la surface exposée à l'atmosphère. Généralement, la cinétique des réactions de polycondensation est extrêmement lente; ces réactions sont donc catalysées par un catalyseur approprié. Comme catalyseurs qui sont utilisés, on fait appel le plus souvent à des catalyseurs à base d'étain, de titane, d'une amine ou des compositions de ces catalyseurs. Les catalyseurs à base d'étain (cf. notamment FR-A-2 557 582) et de titane (cf. notamment FR-A-2 786 497) sont des catalyseurs ayant une bonne efficacité.

Quand aux compositions bicomposantes, elles sont commercialisées et stockées sous la forme de deux composantes, une première composante contenant les matériaux polymériques de base et la deuxième composante contenant le catalyseur. Les deux composantes sont mélangées lors de l'emploi et le mélange réticule sous la forme d'un élastomère relativement dur. Ces compositions à deux composantes sont bien connues et sont notamment décrites dans l'ouvrage de Walter Noll "Chemistry and Technology of Silicones" 1968, 2ème édition aux pages 395 à 398. Ces compositions comportent essentiellement 4 ingrédients différents:
- un polymère réactif **α,ω**-dihydroxydiorganopolysiloxane,
- un agent de réticulation, généralement un silicate ou un polysilicate,
- un catalyseur à l'étain, et
- de l'eau.

Le plus souvent, le catalyseur de condensation est à base d'un composé organique d'étain. En effet, de nombreux catalyseurs à base d'étain ont déjà été proposés comme catalyseur de réticulation de ces RTV-2. Les composés les plus utilisés sont les carboxylates d'alkylétain tels que le monooléate de tributyltain ou les dicarboxylates de dialkylétain tels que le dilaurate de dibutylétain, le diacétate de dibutylétain ou le dilaurate de diméthylétain (voir l'ouvrage de Noll "Chemistry and Technology of silicones" page 337, Academic Press, 1968 - 2ème édition ou les brevets EP 147 323 ou EP 235 049).

Cependant, les catalyseurs à base d'alkylétain, bien que très efficaces, le plus souvent incolores, liquides et solubles dans les huiles silicones présentent l'inconvénient d'être toxiques (CMR2 toxiques pour la reproduction).

Les catalyseurs à base de titane, également très employés dans les RTV-1, présentent cependant un inconvénient majeur: ils ont une cinétique plus lente que les catalyseurs à base d'étain. De plus, ces catalyseurs ne sont pas utilisables dans les RTV-2 du fait de problèmes de gélification.

D'autres catalyseurs sont parfois mentionnés comme les catalyseurs à base de zinc, par exemple décrits dans EP 0354138 A1, zirconium ou d'aluminium, mais ils n'ont connus qu'un faible développement industriel en raison de leur médiocre efficacité.

Pour un développement durable, il apparaît donc nécessaire de développer des catalyseurs non toxiques pour la réaction de polycondensation des organopolysiloxanes.

Un autre aspect important pour un catalyseur de la réaction de polycondensation des organopolysiloxanes est le temps de mise en œuvre (« pot-life » ou « temps de travail »), c'est-à-dire le temps pendant lequel la composition peut être utilisée après mélange sans durcir. Ce temps doit être suffisamment long, pour permettre son utilisation mais suffisamment court pour obtenir un objet moulé manipulable au plus tard quelques minutes ou quelques heures après sa fabrication. Le catalyseur doit donc permettre d'obtenir un bon compromis entre le temps d'utilisation du mélange catalysé et le temps au bout duquel l'objet moulé est manipulable (ces temps dépendent de l'application visée comme par exemple le moulage ou la fabrication de joints). En outre le catalyseur doit conférer au mélange catalysé un temps d'étalement qui ne varie pas en fonction de la durée au stockage.

L'objectif essentiel de la présente invention est donc un procédé permettant aussi bien, à l'humidité de l'air, une réticulation en surface mais aussi une réticulation à cœur la plus complète possible.

Cet objectif est atteint par la présente invention qui concerne tout d'abord un procédé tel que revendiqué requérant une composition organopolysiloxanique, caractérisée en ce qu'elle comprend d'une part une base silicone **B** apte à durcir par réaction de polycondensation en un élastomère silicone et d'autre part, une quantité catalytiquement efficace d'au moins un catalyseur de polycondensation qui est un complexe ou sel métallique **A** de formule **(1):**

[Zn(L¹)(L²)] **(1)**

dans laquelle:
- les symboles L¹ et L² sont identiques ou différents et représentent un ligand qui est un anion β-dicarbonylato ou l'anion énolate d'un composé β-dicarbonylé de formule **(2)** ou un anion acétylacétato dérivé d'un β-cétoester de formule **(2)** suivante:

R¹COCHR²COR³ **(2)**

dans laquelle :
- R¹ représente un radical hydrocarboné linéaire ou ramifié, substitué ou non, en C₁-C₃₀,
- R² est un hydrogène ou un radical hydrocarboné en C₁-C₃₀ linéaire ou ramifié, substitué ou non, de préférence un atome d'hydrogène ou un radical alkyle présentant au plus 4 atomes de carbone ;
- R³ représente un radical hydrocarboné en C₁-C₃₀ linéaire, cyclique ou ramifié, substitué ou non, un aromatique substitué ou non, ou un radical -OR⁴ avec R⁴ représentant un radical hydrocarboné en C₁-C₃₀ linéaire, cyclique ou ramifié, substitué ou non, avec
- R¹ et R² peuvent être reliés pour former un cycle,
- R² et R³ peuvent être reliés pour former un cycle, et
- avec la condition supplémentaire que le complexe ou sel métallique **A** de formule (1) n'est pas le composé diacétylacétonate de zinc ou Zn(acac)₂.

Il est entendu que par "complexe ou sel métallique **A**", cette définition inclut toute forme oligomérique ou analogue dudit complexe ou sel métallique **A.**

Il est du mérite des inventeurs d'avoir trouvé, de manière tout à fait surprenante et inattendue, qu'il convient d'utiliser des complexes métalliques d'une sélection de certains métaux dont au moins un ligand est un anion β-dicarbonylato pour obtenir d'excellents catalyseurs de la réaction de polycondensation d'organopolysiloxanes.

Il est aussi du mérite des inventeurs d'avoir vaincu le préjugé technique qui voulait que, jusqu'alors certains complexes de métaux, comme le zinc par exemple, ne présentent qu'une médiocre activité dans la réaction de polycondensation d'organopolysiloxanes.

La définition des ligands est tirée de l'ouvrage « Chimie Organométallique » de Didier Astruc, publié en 2000 par EDP Sciences, Cf. notamment Chapitre 1, « Les complexes monométalliques », pages 31 et suivantes.

Le catalyseur selon l'invention peut être à l'état solide ou liquide. Il peut être incorporé seul ou dans un solvant approprié. Lorsqu'il est en solvant, une huile silicone peut être additionnée, le solvant est ensuite évaporé de manière à transférer le catalyseur dans un milieu silicone. Le mélange obtenu sert de base catalysante.

Pour la réalisation de l'invention, comme catalyseur de polycondensation selon l'invention, on utilise de préférence un complexe ou sel métallique A choisi parmi le groupe constitué par les complexes (3) à (6) suivants:
(3): Zn(DPM)₂ ou [Zn (*t-Bu-acac*)₂] avec DPM=*(t-Bu-acac)* = l'anion 2,2,6,6-tétraméthyl-3,5-heptanedionato ou l'anion énolate de la 2,2,6,6-tétraméthyl-3,5-heptanedione,
(4): [Zn (*EAA*)₂] avec *EAA* = l'anion éthyle acétoacétato ou l'anion énolate de l'éthyle acétoacétate,
(5): [Zn (*iPr-AA)*₂] avec *iPr-AA* = l'anion isopropyle acétoacétato ou l'anion énolate de l'isopropyle acétoacétate, et
**(6):**

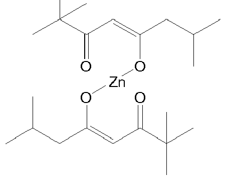

Il est à noter qu'au moins une partie du caractère inventif de l'invention, tient à la sélection judicieuse et avantageuse des associations délimitées de composés métalliques **A** utilisées à titre de catalyseur de polycondensation.

Selon un mode de réalisation préféré, le catalyseur de polycondensation **A** selon l'invention est le bis(2,2,7-triméthyl-3,5-octanedionate) de zinc de formule **(6)**:suivante:

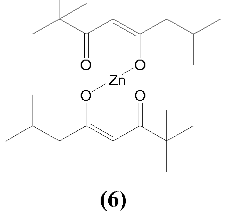

Ce catalyseur présente l'avantage d'être liquide à température ambiante (25°C) et soluble dans des solvants organiques, même dans des alcanes, et dans les huiles silicones.

Selon un mode de réalisation préféré , les ligands β-dicarbonylato L¹ et L² sont des anions β-dicétonato dérivés d'une β-dicétone choisie parmi le groupe constitué par les β-dicétones: hexanedione-2,4; heptanedione-2,4; heptanedione-3,5; l'éthyl-3-pentanedione-2,4; méthyl-5-hexanedione-2,4; octanedione-2,4; octanedione-3,5; diméthyl-5,5-hexanedione-2,4; méthyl-6-heptanedione-2,4; diméthyl-2,2-nonanedione-3,5; diméthyl-2,6 heptanedione-3,5; 2-acétylcyclohexanone *(Cy-acac);* 2,2,6,6-tétraméthyl-3,5-heptanedione (*t-Bu-acac*); 1,1,1,5,5,5-hexafluoro-2,4-pentanedione *(F-acac)*]; benzoylacétone; dibenzoyl-méthane; 3-méthyl-2,4-pentadione; 3-acétyl-pentane-2-one; 3-acétyl-2-hexanone; 3-acétyl-2-heptanone; 3-acétyl-5-méthyl-2-hexanone; stéaroylbenzoylméthane; octanoylbenzoylméthane; 4-t-butyl-4'-méthoxy-dibenzoylméthane; 4,4'-diméthoxy-dibenzoylméthane et 4,4'-di-tert-butyl-dibenzoylméthane.

Selon un autre mode de réalisation préféré, les ligands β-dicarbonylato L¹ et L² sont des anions β-cétoestérato choisis parmi le groupe constitué par les anions dérivés des composés suivants: les esters méthylique, éthylique, n-propylique, isopropylique, n-butylique, sec-butylique, isobutylique, tertiobutylique, isopentylique, n-hexylique, n-octylique, méthyl-1 heptylique, n-nonylique, n-décylique et n-dodécylique de l'acide acétylacétique.

L'invention concerne aussi un procédé utilisant les composés de formules **(5)** et **(6)** suivantes:
**(5):** [Zn (*iPr-AA)*₂] avec *iPr-AA* = l'anion isopropyle acétoacétato ou l'anion énolate de l'isopropyle acétoacétate, et
**(6):** bis(2,2,7-triméthyl-3,5-octanedionate) de zinc de formule:

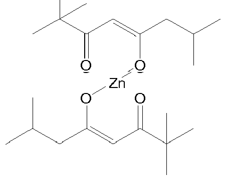

La quantité du catalyseur de polycondensation selon l'invention (complexe ou sel métallique **A)** est comprise entre 0,1 et 10 % en poids de la masse totale, de préférence entre 0,5 et 5 %, que ce soit une préparation mono ou bi-composantes.

### Description de la base silicone B :

Les bases silicone utilisées dans la présente invention réticulant et durcissant par des réactions de polycondensation sont bien connues. Ces bases sont décrites en détail en particulier dans de nombreux brevets et elles sont disponibles dans le commerce.

Ces bases silicones sont mono-composantes, c'est-à-dire conditionnées en un seul emballage, et stables au stockage en l'absence d'humidité, durcissables en présence d'humidité, en particulier d'humidité apportée par l'air ambiant ou par de l'eau générée au sein de la base lors de son emploi.

La base silicone **B** utilisée pour réaliser la composition selon l'invention peut comprendre:
- au moins une huile polyorganosiloxane **C** susceptible de réticuler par polycondensation en un élastomère;
- éventuellement au moins un agent de réticulation **D;**
- éventuellement au moins un promoteur d'adhérence **E;** et
- éventuellement au moins une charge minérale siliceuse, organique et/ou non siliceuse **F.**

L'huile polyorganosiloxane **C** est de préférence un polymère α,ω-dihydroxypolydiorganosiloxane, de viscosité comprise entre 50 et 5 000 000 mPa.s à 25°C et l'agent de réticulation **D** est de préférence un composé organosilicié portant plus de deux groupes hydrolysables liés aux atomes de silicium par molécule. L'huile polyorganosiloxane **C** peut également être fonctionnalisée au niveau de ses extrémités par des radicaux hydrolysables obtenu par condensation d'un précurseur porteur de fonctions hydroxyles avec un silane réticulant porteur de radicaux hydrolysables.

Comme agent de réticulation **(D)** on peut citer :
- les silanes de formule générale suivante :

   R¹ₖSi(OR²)₍₄₋ₖ₎

   dans laquelle les symboles R², identiques ou différents, représentent des radicaux alkyles ayant de 1 à 8 atomes de carbone, tels que les radicaux méthyle, éthyle, propyle, butyle, pentyle, éthyl-2 hexyle, des radicaux oxyalkylènes en C₃-C₆, le symbole R¹ représente un groupe hydrocarboné aliphatique saturé ou insaturé, linéaire ou ramifié, un groupe carbocyclique, saturé ou insaturé et/ou aromatique, monocyclique ou polycyclique et k est égal à 0, 1 ou 2 ; et
- les produits d'hydrolyse partielle de ce silane.

Comme exemple de radicaux alcoxyalkylène en C₃-C₆ on peut citer les radicaux suivants :

CH₃OCH₂CH₂-

CH₃OCH₂CH(CH₃)-

CH₃OCH(CH₃)CH₂-

C₂H₅OCH₂CH₂CH₂-

Le symbole R¹ représente un radical hydrocarboné en C₁-C₁₀ englobant:
- les radicaux alkyles en C₁-C₁₀ tels que les radicaux méthyle, éthyle, propyle, butyle, pentyle, éthyl-2 hexyle, octyle, décyle,
- les radicaux vinyle, allyle, et
- les radicaux cycloalkyles ce C₅-C₈ tels que les radicaux phényle, tolyle et xylyle.

Les agents de réticulation **D** sont des produits accessibles sur le marché des silicones ; de plus leur emploi dans les compositions durcissant dès la température ambiante est connu ; il figure notamment dans les brevets français FR-A-1 126 411, FR-A-1 179 969, FR-A-1 189 216, FR-A-1 198 749, FR-A-1 248 826, FR-A-1 314 649, FR-A-1 423 477, FR-A-1 432 799 et FR-A-2 067 636.

Parmi les agents de réticulation **D,** on préfère plus particulièrement les alkyltrialcoxysilanes, les silicates d'alkyle et les polysilicates d'alkyle, dans lesquels les radicaux organiques sont des radicaux alkylés ayant de 1 à 4 atomes de carbone.

Comme autres exemples d'agents de réticulation **D** qui peuvent être utilisés, on cite plus particulièrement les silanes suivants :
- le propyltriméthoxysilane,
- le méthyltriméthoxysilane,
- l'éthyltriméthoxysilane,
- le vinyltriéthoxysilane,
- le méthyltriéthoxysilane,
- le vinyltriéthoxysilane,
- le propyltriéthoxysilane,
- le tétraéthoxysilane,
- le tétrapropoxysilane,
- le 1,2-bis(triméthoxysilyl)éthane,
- le 1,2-bis(triéthoxysilyl)éthane, et
- le tétraisopropoxysilane,
ou encore: CH₃Si(OCH₃)₃ ; C₂H₅Si(OC₂H₅)₃ ; C₂H₅Si(OCH₃)₃
CH₂=CHSi(OCH₃)₃; CH₂=CHSi(OCH₂CH₂OCH₃)₃
C₆H₅Si(OCH₃)₃ ; [CH₃][OCH(CH₃)CH₂OCH₃]Si[OCH₃]₂
Si(OCH₃)₄ ; Si(OC₂H₅)₄; Si(OCH₂CH₂CH₃)₄; Si(OCH₂CH₂CH₂CH₃)₄
Si(OC₂H₄OCH₃)₄ ; CH₃Si(OC₂H₄OCH₃)₃ ; CICH₂Si(OC₂H₅)₃ ;

Comme autres exemples d'agent de réticulation **D,** on peut citer le polysilicate d'éthyle, ou le polysilicate de n-propyle.

On utilise généralement de 0,1 à 60 parties en poids d'agent de réticulation **D** pour 100 parties en poids de polyorganosiloxane **C** susceptible de réticuler par polycondensation en un élastomère.

Ainsi la composition selon l'invention peut comprendre au moins un promoteur d'adhérence E tel que par exemple les composés organosiliciques portant à la fois :
(1) un ou des groupes hydrolysables liés à l'atome de silicium, et
(2) un ou des groupes organiques substitués par des radicaux comprenant un atome d'azote ou choisis dans le groupe des radicaux (méth)acrylate, époxy, et alcényle, et plus préférentiellement encore dans le groupe constitué par les composés suivants pris seul ou en mélange:
   vinyltriméthoxysilane (VTMO),
   3-glycidoxypropyl-triméthoxysilane (GLYMO),
   méthacryloxypropyltriméthoxysilane (MEMO),
   [H₂N(CH₂)₃]Si(OCH₂CH₂CH₃)₃,
   [H₂N(CH₂)₃]Si(OCH₃)₃
   [H₂N(CH₂)₃]Si(OC₂H₅)₃
   [H₂N(CH₂)₄]Si(OCH₃)₃
   [H₂NCH₂CH(CH₃)CH₂CH₂]SiCH₃(OCH₃)₂
   [H₂NCH₂]Si(OCH₃)₃
   [n-C₄H₉-HN-CH₂]Si(OCH₃)₃
   [H₂N(CH₂)₂NH(CH₂)₃]Si(OCH₃)₃
   [H₂N(CH₂)₂NH(CH₂)₃]Si(OCH2CH₂OCH₃)₃
   [CH₃NH(CH₂)₂NH(CH₂)₃]Si(OCH₃)₃
   [H(NHCH₂CH₂)₂NH(CH₂)₃]Si(OCH₃)₃ 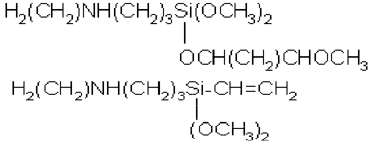 ou des oligomères polyorganosiloxaniques contenant de tels groupes organiques à une teneur supérieure à 20%.

Pour les bases monocomposantes, on utilise comme charges minérales F des produits très finement divisés dont le diamètre particulaire moyen est inférieur à 0,1 µm. Parmi ces charges figurent les silices de combustion et les silices de précipitation; leur surface spécifique BET est généralement supérieure à 40 m²/g. Ces charges peuvent également se présenter sous la forme de produits plus grossièrement divisés, de diamètre particulaire moyen supérieur à 0,1 µm. Comme exemples de telles charges, on peut citer le quartz broyé, les silices de diatomées, le carbonate de calcium, l'argile calcinée, l'oxyde de titane du type rutile, les oxydes de fer, de zinc, de chrome, de zirconium, de magnésium, les différentes formes d'alumine (hydratée ou non), le nitrure de bore, le lithopone, le métaborate de baryum, le sulfate de baryum, les microbilles de verre; leur surface spécifique est généralement inférieure à 30 m²/g.

Ces charges peuvent avoir été modifiées en surface par traitement avec les divers composés organosiliciques habituellement employés pour cet usage. Ainsi ces composés organosiliciques peuvent être des organochlorosilanes, des diorganocyclopolysiloxanes, des hexaorganodisiloxanes, des hexaorganodisilazanes ou des diorganocyclopolysilazanes (brevets français FR-A-1 126 884, FR-A-1 136 885, FR-A-1 236 505, brevet anglais GB-A-1 024 234). Les charges traitées renferment, dans la plupart des cas, de 3 à 30 % de leur poids de composés organosiliciques. Les charges peuvent être constituées d'un mélange de plusieurs types de charges de granulométrie différente; ainsi par exemple, elles peuvent être constituées de 30 à 70 % de silices finement divisées de surface spécifique BET supérieure à 40 m²/g et de 70 à 30 % de silices plus grossièrement divisées de surface spécifique inférieure à 30 m²/g.

L'introduction des charges a pour but de conférer de bonnes caractéristiques mécaniques et rhéologiques aux élastomères découlant du durcissement des compositions conformes à l'invention.

En combinaison avec ces charges peuvent être utilisés des pigments minéraux et/ou organiques ainsi que des agents améliorant la résistance thermique (sels et oxydes de terres rares tels que les oxydes et hydroxydes cériques) et/ou la résistance à la flamme des élastomères. Par exemple on peut utiliser les coktails d'oxyde décrits dans la demande internationale WO 98/29488. Parmi les agents améliorant la résistance à la flamme peuvent être cités les dérivés organiques halogénés, les dérivés organiques du phosphore, les dérivés du platine tels que l'acide chloroplatinique (ses produits de réaction avec des alcanols, des éthers-oxydes), les complexes chlorure platineux-oléfines. Ces pigments et agents représentent ensemble au plus 20 % du poids des charges.

D'autres agents auxiliaires et additifs usuels peuvent être incorporés à la composition selon l'invention; ceux-ci sont choisis en fonction des applications dans lesquelles sont utilisées lesdites compositions.

La base silicone utilisée pour réaliser la composition selon l'invention peut comprendre:
- 100 parties d'huile polyorganosiloxane **C** susceptible de réticuler par polycondensation en un élastomère ;
- 0 à 20 parties d'un agent de réticulation **D;**
- 0 à 20 parties d'un promoteur d'adhérence **E;** et
- 0 à 50 parties de charge **F.**

Outre les constituants principaux, des polymères polyorganosiloxanes linéaires non réactifs **(G)** peuvent être introduits dans le dessein d'agir sur les caractéristiques physiques des compositions conformes à l'invention et/ou sur les propriétés mécaniques des élastomères issus du durcissement de ces compositions.

Ces polymères polyorganosiloxanes linéaires non réactifs **(G)** sont bien connus; ils comprennent plus spécialement: des polymères α,ω-bis(triorganosiloxy)diorganopolysiloxanes de viscosités d'au moins 10 mPa.s à 25°C, formés essentiellement de motifs diorganosiloxy et d'au plus 1 % de motifs monoorganosiloxy et/ou siloxy, les radicaux organiques liés aux atomes de silicium étant choisis parmi les radicaux méthyle, vinyle et phényle, 60 % au moins de ces radicaux organiques étant des radicaux méthyle et 10 % au plus étant des radicaux vinyle. La viscosité de ces polymères peut atteindre plusieurs dizaines de millions de mPa.s à 25°C; ils comprennent donc des huiles d'aspect fluide à visqueux et des gommes molles à dures. Ils sont préparés selon les techniques usuelles décrites plus précisément dans les brevets français FR-A-978 058, FR-A-1 025 150, FR-A-1 108 764, FR-A-1 370 884. On utilise de préférence les huiles α,ω-bis(triméthylsiloxy) diméthylpolysiloxanes de viscosité allant de 10 mPa.s à 1 000 mPa.s à 25°C. Ces polymères qui jouent le rôle de plastifiants peuvent être introduits à raison d'au plus 70 parties, de préférence de 5 à 20 parties, pour 100 parties de d'huile polyorganosiloxane C susceptible de réticuler par polycondensation.

Les compositions selon l'invention peuvent en outre avantageusement comprendre au moins une résine silicone **(H).** Ces résines silicones sont des polymères organopolysiloxanes ramifiés bien connus et disponibles dans le commerce. Elles présentent, par molécule, au moins deux motifs différents choisis parmi ceux de formule R'"₃SiO_{1/2} (motif M), R'"₂SiO_{2/2} (motif D), R"'SiO_{3/2} (motif T) et SiO_{4/2} (motif Q). Les radicaux R'" sont identiques ou différents et sont choisis parmi les radicaux alkyles linéaires ou ramifiés, les radicaux vinyle, phényle, trifluoro-3,3,3 propyle. De préférence, les radicaux alkyles présentent de 1 à 6 atomes de carbone inclus. Plus particulièrement, on peut citer comme radicaux R alkyle, les radicaux méthyle, éthyle, isopropyle, tertiobutyle et n-hexyle. Ces résines sont de préférence hydroxylées et ont dans ce cas une teneur pondérale en groupe hydroxyle comprise entre 5 et 500 meq/100 g.

Comme exemple de résines, on peut citer les résines MQ, les résines MDQ, les résines TD et les résines MDT.

Pour fabriquer les compositions conformes à l'invention, il est nécessaire dans le cas des compositions mono-composantes d'utiliser un appareillage qui permette de mélanger intimement à l'abri de l'humidité, avec et sans apport de chaleur, les divers constituants fondamentaux auxquels sont éventuellement ajoutés les adjuvants et additifs précités. Tous ces ingrédients peuvent être chargés dans l'appareillage selon un ordre quelconque d'introduction. Ainsi il est possible de mélanger tout d'abord les huiles organopolysiloxanes **C** et les charges **F** et d'ajouter ensuite à l'empâtage obtenu les réticulants **D,** les composés **E** et le catalyseur selon l'invention. Il est également possible de mélanger les huiles **C,** les réticulants **D,** les composés **E** et les charges **F** et d'ajouter ultérieurement le catalyseur selon l'invention. Au cours de ces opérations, les mélanges peuvent être chauffés à une température comprise dans l'intervalle 50-180°C sous la pression atmosphérique ou sous une pression réduite afin de favoriser le départ de matières volatiles.

Les compositions mono-composantes, conformes à l'invention, utilisées telles quelles, c'est-à-dire non diluées, ou sous forme de dispersions dans des diluants, sont stables au stockage en l'absence d'eau et durcissent dès les basses températures (après départ des solvants dans le cas des dispersions) en présence d'eau pour former des élastomères.

Après le dépôt des compositions telles quelles, sur des substrats solides, en atmosphère humide, on constate qu'un processus de durcissement en élastomères se met en œuvre, il s'effectue de l'extérieur à l'intérieur de la masse déposée. Une peau se forme d'abord en surface puis la réticulation se poursuit en profondeur. La formation complète de la peau, qui se traduit par un toucher non collant de la surface, demande une période de temps de quelques minutes; cette période dépendant du taux d'humidité relative de l'atmosphère entourant les compositions et de la faculté de réticulation de celles-ci.

Par ailleurs le durcissement en profondeur des couches déposées, qui doit être suffisant pour permettre le démoulage et la manipulation des élastomères formés, nécessite une période de temps plus longue. En effet, cette période dépend non seulement des facteurs cités ci-dessus pour la formation du toucher non collant mais aussi de l'épaisseur des couches déposées, laquelle épaisseur s'échelonne généralement entre 0,5 mm et plusieurs centimètres. Les compositions mono-composantes peuvent être employées pour de multiples applications comme le jointoiement dans l'industrie du bâtiment, l'assemblage des matériaux les plus divers (métaux, matières plastiques, caoutchoucs naturels et synthétiques, bois, carton, faïence, brique, céramique, verre, pierre, béton, éléments de maçonnerie), l'isolation de conducteurs électriques, l'enrobage de circuits électroniques, la préparation de moules servant à la fabrication d'objets en résines ou mousses synthétiques.

Les compositions conformes à l'invention peuvent être employées pour de multiples applications comme le jointoiement et/ou le collage dans l'industrie du bâtiment, l'industrie du transport (exemples : automobile, aérospatiale, ferroviaire, maritime et aéronautique), l'assemblage des matériaux les plus divers (métaux, matières plastiques, caoutchoucs naturels et synthétiques, bois, cartons, polycarbonate, faïence, brique, céramique, verre, pierre, béton et éléments de maçonnerie), l'isolation de conducteurs électriques, l'enrobage de circuits électroniques et la préparation de moules servant à la fabrication d'objets en résines ou mousses synthétiques.

Le procédé de l'invention requiert ainsi une composition polyorganosiloxanique monocomposant stable au stockage en l'absence d'humidité et réticulant, en présence d'eau, en élastomère, caractérisée en ce qu'elle comprend:
- au moins un polyorganopolysiloxane linéaire réticulable présentant des extrémités fonctionnalisées de type alcoxy, oxime, acyle et /ou énoxy, de préférence alcoxy,
- une charge, et
- un catalyseur de la réaction de polycondensation qui est le complexe métallique A selon l'invention et tel que défini ci-dessus.

Des bases mono-composantes sont décrites en détail par exemple dans les brevets EP 141 685, EP 147 323, EP 102 268, EP 21 859, FR 2 121 289 et FR 2 121 631, cités en référence.

A ces bases mono-composantes peuvent être ajoutés des promoteurs d'adhérence E choisis par exemple parmi les composés organosiliciés portant à la fois, d'une part, des groupes organiques substitués par des radicaux choisis dans le groupe des radicaux amino, uréido, isocyanate, époxy, alkènyle, isocyanurate, hydentoile, guanidino et mercaptoester et, d'autre part des groupes hydrolysables, en général des groupes alcoxy liés aux atomes de silicium. Des exemples de tels agents d'adhérence sont décrits dans les brevets américains US 3 517 001, US 4 115 356, US 4 180 642, US 4 273 698, US 4 356 116 et dans les brevets européens EP 31 996 et EP 74 001.

D'autres avantages et caractéristiques de la présente invention apparaîtront à la lecture des exemples suivants donnés à titre illustratif nullement limitatif.

### EXEMPLES

### EXEMPLE 1 : Synthèse des catalyseurs (5) et (6) selon l'invention

### a) Synthèse du catalyseur (5):

[Zn (isopropyle acétoacétato)₂] ou [Zn (*iPr-AA*)₂] avec *iPr-AA* = l'anion isopropyle acétoacétato ou l'anion énolate de l'isopropyle acétoacétate,
Une solution de 100 mmol de méthylate de sodium à 97 % (6.12 g) dans 100 ml d'isopropanol est concentrée par distillation de 20 %, puis 100 mmol d'isopropyle acétoacétate à 95 % (15.93 g) sont ajoutés et la solution est chauffée à 80°C pendant 1h pour obtenir une solution homogène orange. Une solution de chlorure de zinc (50 mmol, 7 g) dans 50 ml d'isopropanol est alors ajoutée à 70°C en 1h. Le chauffage est maintenu entre 80 et 90°C pendant 3h30 puis le chlorure de sodium formé est filtré après refroidissement. La solution alcoolique est évaporée à sec pour conduire à 25.3 g d'une pâte qui est redissoute dans 200 ml d'éthanol. Après filtration à chaud et évaporation à sec, sont obtenus 17.2 g d'un solide blanc (rendement 98%).

Teneur en Zn calculée : 18.59 %, teneur en Zn dosée (ICP) : 18.57 %
Analyse IR (nm): 2989, 1617, 1514, 1246, 1170.

### b) Synthèse du catalyseur bis(2,2,7-triméthyl-3,5-octanedionate) de zinc de formule (6):

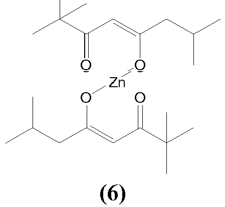

0.86 g d'hydroxyde de sodium (21 mmol) sont dissous dans 10 ml d'éthanol absolu, puis 4 g de 2,2,7-triméthyl-3,5-octanedione (21 mmol) sont ajoutés à la solution obtenue. Une solution de 1.55 g de chlorure de zinc (11 mmol) dans 10 ml d'éthanol absolu est ajoutée en 5 min à la solution jaune pâle de l'énolate de sodium. Le mélange est agité 3h à 20°C puis 10 ml de toluène sont ajoutés. La suspension de chlorure de sodium est refroidie à 10°C sous agitation puis filtrée. Le filtrat est évaporé sous vide pour donner 4 g d'une huile épaisse limpide (rendement 90%).

L'analyse RMN du proton dans le diméthylsulfoxyde (DMSO) montre que la structure du complexe ainsi formé est sous la forme d'un seul isomère : Déplacements chimiques : 0.87 (12H, doublet), 1.05 (18H, singulet), 1.95 (6H, massif), 5.33 (2H, singulet).
Teneur en Zn calculée : 15.1 %, teneur en Zn dosée : (ICP) 14.9 %

### EXEMPLE 2 : Test initial

- Catalyseur **(3):** Zn(DPM)₂ ou [Zn (*t-Bu-acac*)₂] ou avec (*t-Bu-acac*) = l'anion 2,2,6,6-tétraméthyl-3,5-heptanedionato ou l'anion énolate de la 2,2,6,6-tétraméthyl-3,5-heptanedione,
- Catalyseur **(4):** [Zn (*EAA*)₂] avec *EAA* = l'anion éthyle acétoacétato ou l'anion énolate de l'éthyle acétoacétate,
- Catalyseur **(5):** [Zn (*iPr-AA)*₂] avec *iPr-AA* = l'anion isopropyle acétoacétato ou l'anion énolate de l'isopropyle acétoacétate, et
- Catalyseur bis(2,2,7-triméthyl-3,5-octanedionate) de zinc de formule **(6):**

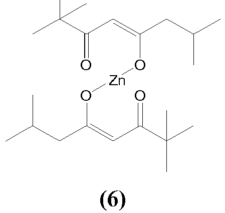

Afin de mettre en évidence l'activité catalytique de nouvelles espèces, deux tests simples ont été mis au point.

Dans les 2 tests, on procède de la façon suivante :
- L'huile fonctionnalisée ou non, puis le catalyseur, puis le réticulant dans le cas du RTV2, puis éventuellement l'eau sont placés successivement dans un petit récipient cylindrique ouvert muni d'un barreau magnétique, et l'agitation est fixée à 300 tours/min. On mesure le temps de l'arrêt de l'agitation qui correspond à une viscosité de 1000 cP (ou mPa) environ, puis le temps où l'huile ne coule plus, le temps de formation d'une peau non collante et le temps de réticulation à cœur. L'activité des nouveaux catalyseurs est comparée à celle du dilaurate de tétra butyldistannoxane ou Tegokat 225 l'un des plus rapides catalyseurs de type dialkylétain (1.24 mmol en équivalent Sn).

### Test dit RTV1 :

La même huile utilisée précédemment est fonctionnalisée préalablement par le vinyltriméthoxysilane (VTMO) ; l'espèce à tester est mise en contact avec cette huile dans les mêmes conditions que précédemment, puis 2 équivalents d'eau sont ajoutés (2 éq./OH initiaux).

Les quantités utilisées dans les exemples ci-dessous sauf mention sont les suivantes :
- 4.77 g d'huile 48V100 fonctionnalisée VTMO,
- 1.24 mmol d'espèce à tester (1/2 éq./OH)
- 90 µl d'eau (ajoutés après 1 min d'agitation = t₀).

### Test dit RTV2 :

L'espèce à tester est mise en contact avec une huile polydiméthylsiloxanique α,ω-dihydroxylée courte (½ équivalent par rapport à la teneur en OH, viscosité de 100 mPa.s, huile 48V100) puis un réticulant, le silicate d'éthyle est ajouté (1 équivalent /OH), ou le même volume de silicate d'éthyle dit « avancé », c'est-à-dire un mélange d'éthoxypolysiloxanes (dans ce cas > léq/OH).

Les quantités utilisées dans les exemples ci-dessous sauf mention sont les suivantes :
- 4.48 g d'huile 48V100 à 0.553 mmol OH/g (viscosité 100 cP ou mPa)
- 1.24 mmol d'espèce à tester (1/2 éq./OH)
- 0.52 g de silicate d'éthyle (1 éq./OH) ou le même volume de silicate « avancé » que le silicate d'éthyle (= 0.82 g)

Les résultats du test RTV1 sont indiqués dans le tableau I ci-dessous :

**Tableau I: Tests RTV1**

| | métal | catalyseur | Temps arrêt agitation (h:min) | Temps fin coulabilité (h:min) | Temps touché non collant (h:min) | Temps réticulation (h:min) | Dur/liquide après 24h |
|---|---|---|---|---|---|---|---|
| **Comparatif 1** | **Sn** | Tegokat 225 | 00:19 | 00:22 | 00:25 | 00:34 | Dur |
| **Comparatif 2** | **Zn** | Zn (éthyl-2-hexanoate)₂ | 30h | | | Pas de réticulation | liquide |
| **Catalyseur (3) invention** | **Zn** | Zn(DPM)₂ | 01:00 | 01:10 | 01:45 | 01:30 | Très dur |

Les résultats du test RTV2, réticulant silicate d'éthyle, sont indiqués dans le tableau II ci-dessous :

**Tableau II: Tests RTV2 silicate d'éthyle**

| | métal | catalyseur | Temps arrêt agitation (h:min) | Temps fin coulabilité (h:min) | Temps touché non collant (h:min) | Temps réticulation (h:min) | Dur/liquide après 24h |
|---|---|---|---|---|---|---|---|
| Comparatif 1 | **Sn** | Dibutyllauratedistannoxane (Tegokat225) | 00:20 | 00:30 | 00:42 | 00:42 | dur |
| Comparatif 2 | **Zn** | Zn(acac)₂, H₂O | 03:15 | 04:00 | 04:20 | **entre 7 et 24h** | dur |
| Comparatif 3 | **Zn** | Zn(2-éthylhexanoate)₂ | 30h | | | Pas de réticulation | Liquide visqueux |
| Catalyseur **(3)** Invention | **Zn** | Zn(DPM)₂ | 02:20 | 02:20 | 02:20 | 03:20 | dur |
| Catalyseur **(4)** Invention | **Zn** | [Zn (*EAA*)₂] | 03:40 | 04:35 | 04:35 | 04:35 | dur |
| Catalyseur **(5)** Invention | **Zn** | [Zn (*iPr-AA*)₂] | 04:00 | 04:30 | 04:30 | peau en surface | dur |

Les tests comparatifs 2 et 3 (diacétylacétonate de zinc ou [Zn(acac)₂, H₂O] et Zn(2-éthylhexanoate)₂] montrent que les catalyseurs selon l'art antérieur ne permettent pas une réticulation dans des temps acceptables pour ce type d'application.

Les résultats du test RTV2, réticulant silicate "avancé", sont indiqués dans le tableau III ci-dessous.

**Tableau III: Tests RTV2 silicate avancé"**

| | métal | catalyseur | Temps arrêt agitation (h:min) | Temps fin coulabilité (h:min) | Temps touché non collant (h:min) | Temps réticulation (h:min) | Dur/liquide après 24h |
|---|---|---|---|---|---|---|---|
| Comparatif 1 | **Zn** | Zn (2-éthylhexanoate)₂ | | | | Pas de réticulation | Liquide |
| Catalyseur **(3)** Invention | **Zn** | Zn(DPM)₂ | 00:45 | 00:45 | 00:45 | 01:00 | très dur |
| Catalyseur **(5)** Invention | **Zn** | [Zn (*iPr-AA*)₂] | 01:50 | 02:00 | 02:00 | 03:00 | dur |

### Exemple 3: Test en empâtage pour RTV1 et RTV2

Dans un second temps, certains catalyseurs sont aussi testés dans des systèmes plus proches appelés "empâtages".

En **RTV1,** l'empâtage utilisé est préparé comme suit : à un mélange de 3464 g d'une huile α,ω-dihydroxylée de viscosité de 20000 centipoises contenant 0.066% d'OH, et de 120 g de vinyltriméthoxysilane sous agitation est ajouté 16 g d'une solution de lithine à 2 % en poids dans le méthanol, puis après 5 min 400 g de silice de pyrogénation AE55 sont ajoutés. Le mélange est dévolatilisé sous vide puis stocké à l'abri de l'humidité.

Pour chaque test, le catalyseur testé est mélangé à 50 g de cet empâtage, puis le potentiel catalytique est évalué de 3 façons (voir les tableaux de résultats ci- après) :
- le temps de formation de peau (TFP), temps au bout duquel on observe une réticulation en surface, sur film de 2 mm,
- la persistance d'un toucher collant à 48h,
- la dureté (en Shore A) d'un cordon de 6 mm d'épaisseur dans de conditions régulées (23°C et 50% d'humidité relative) et sur des temps croissants (2, 3, 4,7 et 14 jours). La stabilité à haute température est aussi évaluée par des mesures de dureté réalisées sur le cordon après 7 jours à température ambiante suivis de 7 jours à 100°C.
   Nota : La dureté Shore est mesurée sur un cordon de 6 mm. Dans les tableaux de résultats, le symbole « > » correspond aux valeurs de dureté mesurées sur la partie supérieure du cordon et le symbole « < » correspond aux valeurs de dureté mesurées sur la partie inférieure du cordon moins exposée à l'air ambiant que la partie supérieure.

Différents catalyseurs selon l'invention ont été testés.

A titre comparatif, comme précédemment, ont été également testés:
- un catalyseur à base d'étain: le dilaurate de dibutylétain (DLDBE).

Les résultats sont indiqués dans le tableau IV ci-dessous:

**Tableau IV : tests en empâtages RTV1**

| **Catalyseur** | **solvant en %** | **%poids** | **TFP bâton** | **Toucher collant** | **Dureté Shore A sur 6mm** | | | | | |
|---|---|---|---|---|---|---|---|---|---|---|
| | | | | **à** | **2J** | **3J** | **4J** | **7J** | **14J** | **7J TA + 7J 100°C** |
| | | | **minutes** | **48h** | **> <** | **> <** | **> <** | **> <** | **> <** | **> <** |
| DLDBE Comparatif | / | 1 | 10 | non | 32 22 | | | 32 29 | | |
| Catalyseur **(3)** Invention Zn(DPM)₂ | acétate d'ethyle 50% | 2,5 | 90 | oui | 9 14 | 15 19 | 17 20 | 21 22 | 24 22 | 24 22 |

En **RTV2,** les tests sont réalisés directement sur un mélange d'une huile visqueuse polydiméthylsiloxane α,ω-dihydroxylée (de viscosité 14000 mPa.s) et de réticulant silicate avancé (1 g pour 22.5 g d'huile) auquel on ajoute et mélange le catalyseur. Le temps de travail ou "pot-life" est d'abord mesuré (temps au delà duquel la viscosité du mélange empêche son utilisation), puis à partir d'un autre mélange, un pion de 6 mm d'épaisseur est coulé pour les mesures de dureté dans le temps (réalisées comme précédemment en dessus et en dessous).

A titre comparatif, comme précédemment, ont été également testés:
- un catalyseur à base d'étain: le dinéodécanoate de diméthylétain (UL28).

Les résultats sont indiqués dans le tableau V ci-dessous.

**Tableau V- Tests en empâtages RTV2**

| catalyseur | Solvant | Equivalent molaire | % poid | pot-life (min) | Dureté Shore A sur 6 mm | | | | | |
|---|---|---|---|---|---|---|---|---|---|---|
| | | | | | 1J | | 4J | | 21J | |
| | | | | | > | < | > | < | > | < |
| UL28 Comparatif | non | 1 | 1,4 | 15 | 24 | 19 | 25 | 25 | 23 | 23 |
| Catalyseur **(3)** Invention Zn(DPM)₂ | AcOEt | 3 | 3,6 | 30 | 19 | 11 | 22 | 23 | 23 | 25 |
| | Pas de solvant mais sous forme broyée | 3 | 3,7 | 9 | 21 | 10 | 23 | 23 | 24 | 24 |

Dans le tableau ci-dessus, le symbole « > » correspond aux valeurs de dureté mesurées sur la partie supérieure du pion et le symbole « < » correspond aux valeurs de dureté mesurées sur la partie inférieure du pion, moins exposée à l'air ambiant que la partie supérieure.

### EXEMPLE 4 : Tests en empâtages RTV2: Catalyseur (6)

Les essais ont été réalisés en mélangeant 25 g d'huile α,ω-dihydroxylée de viscosité 14000 m.Pa.s, 1.06 g de réticulant silicate d'éthyle prépolymérisé et x mmol du catalyseur **(6)** selon l'invention qui présente l'avantage de se présenter sous une forme liquide et visqueuse et soluble dans les silicones. Les résultats sont comparés avec ceux obtenus avec le catalyseur bis-néodécanoate de diméthylétain.

On mesure le temps de travail, c'est-à-dire le temps nécessaire à la formation d'un gel qui n'est plus facilement coulable et les duretés Shore A (dessus et dessous) d'un pion démoulé d'épaisseur 6 mm.

**Tableau VI**

| Catalyseur testé | mmol/25g d'huile | Temps de travail (min) | DSA 1J | | DSA 4 jours | | DSA 7 jours | |
|---|---|---|---|---|---|---|---|---|
| | | | > | < | > | < | > | < |
| Catalyseur **(6)** [Invention] | 2 | 12 | 19 | 9 | 23 | 23 | 23 | 26 |
| Catalyseur **(6)** [Invention] | 1 | 18 | 16 | 11 | 20 | 21 | 19 | 23 |
| bis-néodécanoate de diméthylétain [Comparatif] | 0.7 | 6 | 22.5 | 22.5 | / | | / | |

Dans le tableau ci-dessus, le symbole « > » correspond aux valeurs de dureté mesurées sur la partie supérieure du pion et le symbole « < » correspond aux valeurs de dureté mesurées sur la partie inférieure du pion, moins exposée à l'air ambiant que la partie supérieure

**Remarque** : le temps de travail avec le nouveau catalyseur (6) selon l'invention est un peu plus long que dans le cas du catalyseur étain permettant une bonne flexibilité d'utilisation pour une application de type RTV2.

## Revendications

1. Procédé de préparation d'un élastomère constitué d'une étape de réticulation et durcissement dès la température ambiante d'une composition polyorganosiloxanique monocomposante stable au stockage en l'absence d'humidité, ne contenant pas de catalyseurs à base d'alkylétain qui présentent des problèmes de toxicité et réticulant, en présence d'eau, en élastomère, **caractérisée en ce qu'**elle comprend :
- au moins un polyorganopolysiloxane linéaire réticulable présentant des extrémités fonctionnalisées de type alcoxy, oxime, acyle et /ou énoxy, de préférence alcoxy,
- au moins une charge, et
- une quantité catalytiquement efficace d'au moins un catalyseur de la réaction de polycondensation qui est le complexe ou sel métallique **A** de formule **(1):**
[Zn(L¹)(L²)] **(1)**
dans laquelle:
- les symboles L¹ et L² sont identiques ou différents et représentent un ligand qui est un anion β-dicarbonylato ou l'anion énolate d'un composé β-dicarbonylé de formule **(2)** ou un anion acétylacétato dérivé d'un β-cétoester de formule **(2)** suivante:
R¹COCHR²OR³ **(2)**
dans laquelle :
- R¹ représente un radical hydrocarboné linéaire ou ramifié, substitué ou non, en C₁-C₃₀,
- R² est un hydrogène ou un radical hydrocarboné en C₁-C₃₀ linéaire ou ramifié, substitué ou non, de préférence un atome d'hydrogène ou un radical alkyle présentant au plus 4 atomes de carbone ;
- R³ représente un radical hydrocarboné en C₁-C₃₀ linéaire, cyclique ou ramifié, substitué ou non, un aromatique substitué ou non, ou un radical -OR⁴ avec R⁴ représentant un radical hydrocarboné en C₁-C₃₀ linéaire, cyclique ou ramifié, substitué ou non, avec
- R¹ et R² peuvent être reliés pour former un cycle,
- R² et R³ peuvent être reliés pour former un cycle, et
- avec la condition supplémentaire que le complexe ou sel métallique **A** de formule **(1)** n'est pas le composé diacétylacétonate de zinc ou Zn(acac)₂.

2. Procédé selon la revendication 1 **caractérisé en ce que** le complexe ou sel métallique **A** est choisi parmi le groupe constitué par les complexes **(3)** à **(6)** suivants:
**(3):** Zn(DPM)₂ ou [Zn (*t-Bu-acac*)*₂*] avec DPM=*(t-Bu-acac)* = l'anion 2,2,6,6-tétraméthyl-3,5-heptanedionato ou l'anion énolate de la 2,2,6,6-tétraméthyl-3,5-heptanedione,
**(4):** [Zn (*EAA*)₂] avec *EAA* = l'anion éthyle acétoacétato ou l'anion énolate de l'éthyle acétoacétate,
**(5):** [Zn (*iPr-AA*)₂] avec *iPr-AA* = l'anion isopropyle acétoacétato ou l'anion énolate de l'isopropyle acétoacétate, et
**(6):**

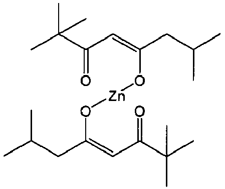

3. Procédé selon la revendication 1 **caractérisé en ce que** les ligands β-dicarbonylato L¹ et L² sont des anions β-dicétonato dérivés d'une β-dicétone choisie parmi le groupe constitué par les β-dicétones: hexanedione-2,4; heptanedione-2,4; heptanedione-3,5; l'éthyl-3-pentanedione-2,4; méthyl-5-hexanedione-2,4; octanedione-2,4; octanedione-3,5; diméthyl-5,5-hexanedione-2,4; méthyl-6-heptanedione-2,4; diméthyl-2,2-nonanedione-3,5; diméthyl-2,6 heptanedione-3,5; 2-acétylcyclohexanone (*Cy-acac*); 2,2,6,6-tétraméthyl-3,5-heptanedione *(t-Bu-acac);* 1,1,1,5,5,5-hexafluoro-2,4-pentanedione (*F-acac*)]; benzoylacétone; dibenzoyl-méthane; 3-méthyl-2,4-pentadione; 3-acétyl-pentane-2-one; 3-acétyl-2-hexanone; 3-acétyl-2-heptanone; 3-acétyl-5-méthyl-2-hexanone; stéaroylbenzoylméthane; octanoylbenzoylméthane; 4-t-butyl-4'-méthoxy-dibenzoylméthane; 4,4'-diméthoxy-dibenzoylméthane et 4,4'-di-tert-butyl-dibenzoylméthane.

4. Procédé selon la revendication 1 **caractérisé en ce que** les ligand β-dicarbonylato L¹ et L² sont des anions β-cétoestérato choisis parmi le groupe constitué par les anions dérivés des composés suivants: les esters méthylique, éthylique, n-propylique, isopropylique, n-butylique, sec-butylique, isobutylique, tertiobutylique, isopentylique, n-hexylique, n-octylique, méthyl-1 heptylique, n-nonylique, n-décylique et n-dodécylique de l'acide acétylacétique.

## Patentansprüche

1. Verfahren zur Herstellung eines Elastomers, das aus einem Vernetzungs- und Härtungsschritt einer einkomponentigen Polyorganosiloxanzusammensetzung, die in Abwesenheit von Feuchtigkeit lagerstabil ist, keine Toxizitätsprobleme aufweisenden Katalysatoren auf Alkylzinn-Basis enthält und in Gegenwart von Wasser zu einem Elastomer vernetzt, ab Umgebungstemperatur, besteht, **dadurch gekennzeichnet, dass** die Zusammensetzung Folgendes umfasst:
- mindestens ein vernetzbares lineares Polyorganopolysiloxan mit funktionalisierten Enden vom Alkoxy-, Oxim-, Acyl- und/oder Enoxy-Typ, vorzugsweise vom Alkoxy-Typ,
- mindestens einen Füllstoff und
- eine katalytisch wirksame Menge mindestens eines Katalysators für die Polykondensationsreaktion, bei dem es sich um einen Metallkomplex oder ein Metallsalz **A** der Formel **(1)** handelt:
[Zn(L¹)(L²)] **(1)**,
wobei:
- die Symbole L¹ und L² gleich oder verschieden sind und für einen Liganden stehen, bei dem es sich um ein β-Dicarbonylato-Anion oder das Enolat-Anion einer β-Dicarbonylverbindung der Formel **(2)** oder ein Acetylacetato-Anion, das sich von einem β-Ketoester der folgenden Formel (2) ableitet, handelt:
R¹COCHR²COR³ (**2**),
wobei:
- R¹ für einen substituierten oder unsubstituierten, linearen oder verzweigten C₁-C₃₀-Kohlenwasserstoffrest steht,
- R² für Wasserstoff oder einen substituierten oder unsubstituierten, linearen oder verzweigten C₁-C₃₀-Kohlenwasserstoffrest, vorzugsweise ein Wasserstoffatom oder einen Alkylrest mit höchstens 4 Kohlenstoffatomen, steht,
- R³ für einen substituierten oder unsubstituierten, linearen, cyclischen oder verzweigten C₁-C₃₀-Kohlenwasserstoffrest, einen substituierten oder unsubstituierten aromatischen Rest oder einen -OR⁴-Rest steht, wobei R⁴ für einen substituierten oder unsubstituierten, linearen, cyclischen oder verzweigten C₁-C₃₀-Kohlenwasserstoffrest steht, wobei
- R¹ und R² zu einem Ring verbunden sein können,
- R² und R³ zu einem Ring verbunden sein können, und
- mit der zusätzlichen Maßgabe, dass es sich bei dem Metallkomplex bzw. Metallsalz **A** der Formel **(1)** nicht um die Verbindung Zinkdiacetylacetonat oder Zn(acac)₂ handelt.

2. Verfahren nach Anspruch 1, **dadurch gekennzeichnet, dass** der Metallkomplex bzw. das Metallsalz **A** aus der Gruppe bestehend aus den folgenden Komplexen **(3)** bis **(6)** ausgewählt ist:
(**3**): Zn(DPM)₂ oder [Zn(*t-Bu-acaC)*₂] mit DPM = (t-*Bu-acac)* = das 2,2,6,6-Tetramethyl-3,5-heptan-dionato-Anion oder das Enolat-Anion von 2,2,6,6-Tetramethyl-3,5-heptandion,
(**4**): [Zn(*EAA*)₂] mit *EAA* = das Ethylacetoacetato-Anion oder das Enolat-Anion von Ethylacetoacetat,
**(5):** [Zn (*iPr-AA*)₂] mit *iPr-AA* = das Isopropylacetoacetat-Anion oder das Enolat-Anion von Isopropylacetoacetat, und
(**6**):

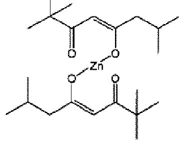

3. Verfahren nach Anspruch 1, **dadurch gekennzeichnet, dass** es sich bei den β-Dicarbonylato-Liganden L¹ und L² um β-Diketonato-Anionen handelt, die sich von einem β-Diketon ableiten, das aus der Gruppe bestehend aus den folgenden β-Diketonen ausgewählt ist: 2,4-Hexandion; 2,4-Heptandion; 3,5-Heptandion; 3-Ethyl-2,4-pentandion; 5-Methyl-2,4-hexandion; 2,4-Octandion; 3,5-Octandion; 5,5-Dimethyl-2,4-hexandion; 6-Methyl-2,4-heptandion; 2,2-Dimethyl-3,5-nonandion; 2,6-Dimethyl-3,5-heptandion; 2-Acetylcyclohexanon *(Cy-acac);* 2,2,6,6-Tetramethyl-3,5-heptandion *(t-Bu-acac);* 1,1,1,5,5,5-Hexafluor-2,4-pentandion *(F-acac);* Benzoylaceton; Dibenzoylmethan; 3-Methyl-2,4-pentandion; 3-Acetyl-2-pentanon; 3-Acetyl-2-hexanon; 3-Acetyl-2-heptanon; 3-Acetyl-5-methyl-2-hexanon; Stearylbenzoylmethan; Octanoylbenzoylmethan; 4-(t-Butyl)-4'-Methoxydibenzoylmethan; 4,4'-Dimethoxydibenzoylmethan und 4,4'-Di(tert-butyl)dibenzoylmethan.

4. Verfahren nach Anspruch 1, **dadurch gekennzeichnet, dass** es sich bei den β-Dicarbonylato-Liganden L¹ und L² um β-Ketoesterato-Anionen handelt, die aus der Gruppe bestehend aus denjenigen Anionen ausgewählt sind, die sich von den folgenden Verbindungen ableiten: den Methyl-, Ethyl-, n-Propyl-, Isopropyl-, n-Butyl-, sec-Butyl-, Isobutyl-, tert-Butyl-, Isopentyl-, n-Hexyl-, n-Octyl-, 1-Methylheptyl-, n-Nonyl-, n-Decyl- und n-Dodecylestern von Acetylessigsäure.

## Claims

1. Process for the preparation of an elastomer consisting of a stage of crosslinking and curing, at ambient temperature, of a single-component polyorganosiloxane composition which is stable on storage in the absence of moisture, which does not contain alkyltin-based catalysts which exhibit problems of toxicity and which crosslinks, in the presence of water, to give an elastomer, **characterized in that** it comprises:
- at least one crosslinkable linear polyorganosiloxane exhibiting functionalized ends of alkoxy, oxime, acyl and/or enoxy, preferably alkoxy, type,
- at least one filler, and
- a catalytically effective amount of at least one catalyst of the polycondensation reaction which is the metal complex or salt **A** of formula (**1**):
[Zn(L¹)(L²)] (**1**)
in which:
- the symbols L¹ and L² are identical or different and represent a ligand which is a β-dicarbonylato anion or the enolate anion of a β-dicarbonyl compound of formula (**2**) or an acetylaceto anion derived from a β-ketoester of following formula (**2**):
R¹COCHR²COR³ (**2**)
in which
- R¹ represents a substituted or unsubstituted and linear or branched C₁-C₃₀ hydrocarbon radical,
- R² is a hydrogen or a substituted or unsubstituted and linear or branched C₁-C₅₀ hydrocarbon radial, preferably a hydrogen atom or an alkyl radical exhibiting at most 4 carbon atoms;
- R³ represents a linear, cyclic or branched C₁-C₃₀ hydrocarbon radical which is substituted or unsubstituted, a substituted or unsubstituted aromatic or an -OR⁴ radical with R⁴ representing a substituted or unsubstituted and linear, cyclic or branched C₁-C₃₀ hydrocarbon radical, with
- R¹ and R² being able to be connected to form a ring,
- R² and R³ being able to be connected to form a ring, and
- with the additional condition that the metal complex or salt **A** of formula (**1**) is not the compound zinc diacetylacetonate or Zn(acac)₂.

2. Process according to Claim 1, **characterized in that** the metal complex or salt **A** is chosen from the group consisting of the following complexes (**3**) to (**6**):
(**3**): Zn(DPM)₂ or [Zn (*t-Bu-acac*)₂] with DPM = (t-Bu-*acac*) = the 2,2,6,6-tetramethyl-3,5-heptanedionato anion or the enolate anion of 2,2,6,6-tetramethyl-3,5-heptanedione,
(**4**): [Zn (*EAA*)₂] with *EAA* = the ethyl acetoacetato anion or the enolate anion of ethyl acetoacetate,
(**5**): [Zn (*iPr-AA*)₂] with *iPr-AA* = the isopropyl acetoacetato anion or the enolate anion of isopropyl acetoacetate, and
(**6**) :

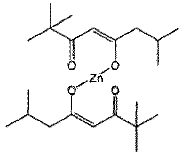

3. Process according to Claim 1, **characterized in that** the β-dicarbonylato ligands L¹ and L² are β-diketonato anions derived from a β-diketone chosen from the group consisting of the β-diketones: 2,4-hexanedione; 2,4-heptadione; 3,5-heptanedione; 3-ethyl-2,4-pentanedione; 5-methyl-2,4-hexanedione; 2,4-octanedione; 3,5-octanedione; 5,5-dimethyl-2,4-hexanedione; 6-methyl-2,4-heptanedione; 2,2-dimethyl-3,5-nonanedione; 2,6-dimethyl-3,5-heptanedione; 2-acetylcyclohexanone *(Cy-acac);* 2,2,6,6-tetramethyl-3,5-heptanedione *(t-Bu-acac);* 1,1,1,5,5,5-hexafluoro-2,4-pentanedione (F-acac); benzoylacetone; dibenzoylmethane; 3-methyl-2,4-pentanedione; 3-acetyl-2-pentanone; 3-acetyl-2-hexanone; 3-acetyl-2-heptanone; 3-acetyl-5-methyl-2-hexanone; stearylbenzoylmethane; octanoylbenzoylmethane; 4-(t-butyl)-4'-methoxydibenzoylmethane; 4,4'-dimethoxy-dibenzoylmethane and 4,4'-di(tert-butyl)dibenzoylmethane.

4. Process according to Claim 1, **characterized in that** the β-dicarbonylato ligands L¹ and L² are β-ketoesterato anions chosen from the group consisting of the anions derived from the following compounds: the methyl, ethyl, n-propyl, isopropyl, n-butyl, sec-butyl, isobutyl, tert-butyl, isopentyl, n-hexyl, n-octyl, 1-methylheptyl, n-nonyl, n-decyl and n-dodecyl esters of acetylacetic acid.
